(19) Europäisches Patentamt
European Patent Office
Office européen des brevets

(11) Numéro de publication: **0 207 845**
**A1**

(12) # DEMANDE DE BREVET EUROPEEN

(21) Numéro de dépôt: 86401320.6

(22) Date de dépôt: 17.06.86

(51) Int. Cl.⁴: **C 07 D 471/04**
C 07 D 491/04, C 07 D 215/56
A 61 K 31/435, A 61 K 31/47
A 61 K 31/495
//(C07D471/04, 221:00, 221:00),
(C07D491/04, 317:00, 221:00)

(30) Priorité: 20.06.85 FR 8509371

(43) Date de publication de la demande:
07.01.87 Bulletin 87/2

(84) Etats contractants désignés:
AT BE CH DE FR GB IT LI LU NL SE

(71) Demandeur: **S.A. PANMEDICA Société dite:**
**Zone Industrielle - Ilôt J**
**F-06516 Carros(FR)**

(72) Inventeur: **Laruelle, Claude**
**Avenue Bellevue**
**F-06270 Villeneuve Loubert(FR)**

(72) Inventeur: **Lepant, Marcel**
**7 rue Mellarède**
**F-06100 Nice(FR)**

(72) Inventeur: **Raynier, Bernard**
**9 Chemin du Malvan**
**F-06800 Cagnes(FR)**

(74) Mandataire: **Orès, Bernard et al,**
**Cabinet ORES 6, avenue de Messine**
**F-75008 Paris(FR)**

(54) **Nouveaux antibactériens contenant des dérivés des acides oxo-4 pyridine carboxylique et leur procédé de préparation.**

(57) La présente invention est relative à de nouveaux antibactériens, ils répondent à la formule générale I

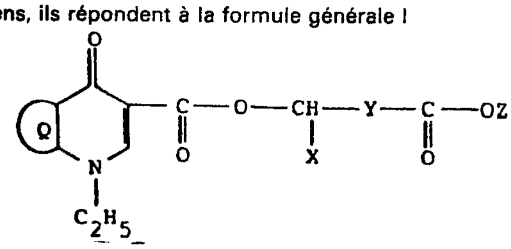

(I)

dans laquelle :

Q représente un cycle aromatique,

X représente un atome d'hydrogène ou un groupe alkyle linéaire ou ramifié,

Y représente une liaison ou un groupe alkyle,

Z représente un atome d'hydrogène, un groupe alkyle, aralkyle ou hétéroalkyle.

Antibactériens à action pharmacologique multiple.

EP 0 207 845 A1

La présente invention est relative à de nouveaux antibactériens à action pharmacologique multiple et à leur procédé de préparation.

La recherche d'agents antibactériens a conduit à la découverte d'une grande variété de structures apparentées aux acides dihydro-1,4 alkyl-1 oxo-4 pyridine carboxyliques-3.

Ainsi, DAINIPPON Pharmaceutical dans son brevet britannique 1.129.358 (1966) décrit les propriétés antibactériennes intéressantes de dérivés de formule générale:

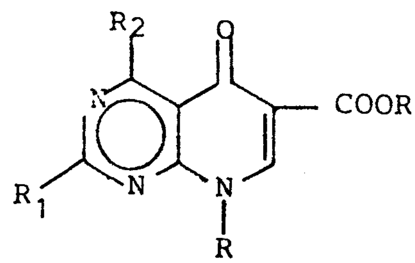

comprenant entre autres l'acide PIROMIDIQUE quand $R_1$ = pyrrolidine.

Le même déposant décrit , dans son brevet français 2.103.618 (1971) des propriétés antibactériennes bien supérieures quand le cycle pyrrolidine cité précédemment est substitué. A partir de la même structure fondamentale, DAINIPPON revendique dans son brevet français 2.196.159 (1973) la structure générale :

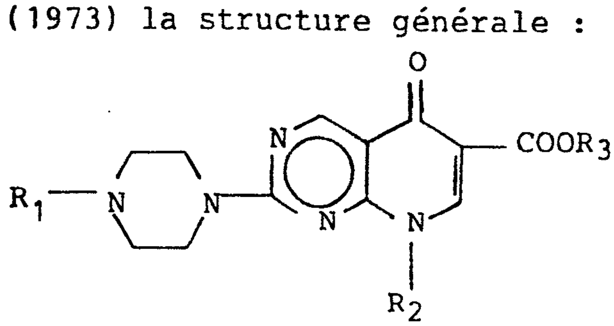

dont les activités bactéricides sont plus intéressantes que celles des homologues décrits précédemment. Un peu plus tard, R. BELLON dans son brevet français 2.364.920 (1977) revendique les dérivés selon la formule générale ci-dessus attribuant au radical $R_1$, des significations

non précédemment revendiquées, à savoir des radicaux carbonylés, alors que dans le même temps DAINIPPON dans son brevet français 2.359.140 (1977) revendiquait cette même formule générale mais où $R_1$ = $NH_2$-phényl-$CH_2$- entrainant une activité antibactérienne tout à fait satisfaisante aussi bien in vitro que in vivo contre les bactéries gram positives et gram négatives.

Dans cette même série des acides alkyl-1 pyridone-4 carboxyliques-3, l'acide NALIDIXIQUE faisait l'objet du dépôt français 1.427.608 (1962) de STERLING DRUG selon la formule générale :

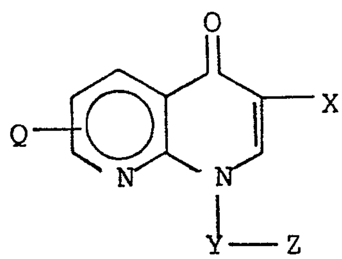

où X représente un groupe carboxy, carbalkoxy, carbamyle.

A partir de cette structure, YAMANOUCHI réalisait l'amide de l'ampicilline (B. Fr. 2.183.895) et FUJIMOTO celui de la cephalexine (B. Jap. 82.46990).

Cette structure générale est affinée par R. BELLON B. Fr. 2.500.833 qui revendique les dérivés de formule générale :

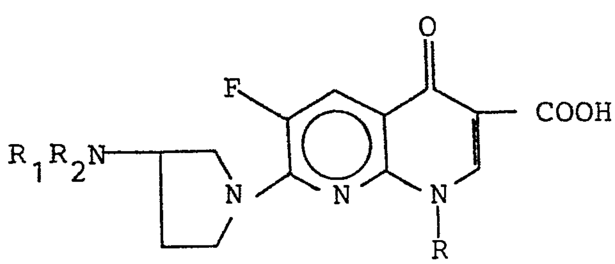

(IV)

pour ses activités bactéricides notamment vis-à-vis des gram.

La série des acides dihydro-1,4 éthyl-1 oxo-4 pyridine carboxyliques détermine avec un noyau aromatique condensé une quinolone dont l'acide oxolinique :

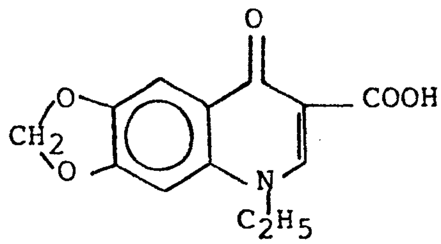

représente la tête de série (B. Fr. M 4148 WARNER LAMBERT). Cette structure quinolone carboxylique 3 était affinée progressivement pour aboutir à la PERFLOXACINE.

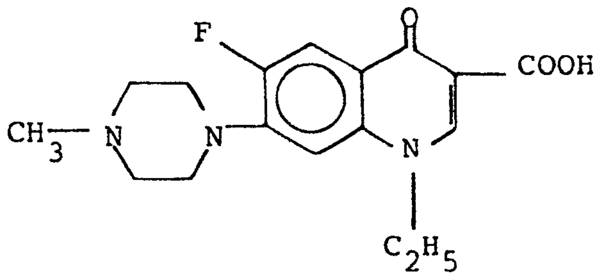

de Roger BELLON.
ou à la ROSOXACINE de STERLING DRUG (US Pat. 3.753.993).

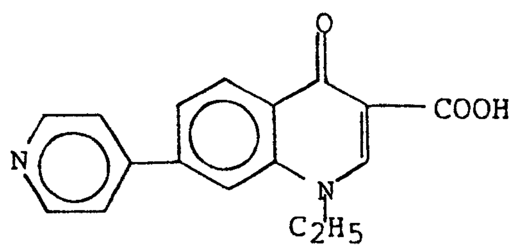

ou encore à la NORFLOXACINE (US Pat. 4.292.317).

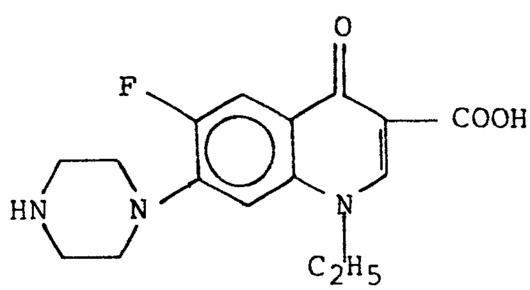

On voit donc se préciser dans le temps des structures relatives à une même structure de base et dont les qualités phármacologiques notamment antibactériennes vont croissant.

Les dérivés de l'acide oxolinique ou nalidixique, l'ENOXACINE, la PERFLOXACINE, la NORFLOXACINE, la ROSOXACINE ou les dérivés de l'acide pipémidique sont des antibactériens très appréciés et largement utilisés.

La présente invention s'est fixée pour but de pourvoir à une nouvelle famille d'antibactériens englobant les dérivés des produits ci-dessus nommés et bien d'autres encore. Elle a en outre pour but d'élargir le spectre antibactérien de ces produits et d'ajouter d'autres actions pharmacologiques à leur activité antibactérienne.

La présente invention a pour objet des composés répondant à la formule générale (I) ci-après :

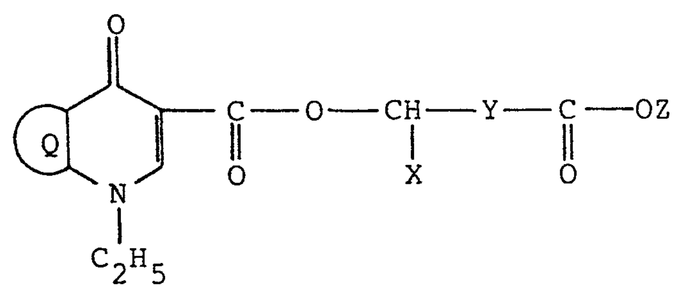

(I)

dans laquelle, . Q représente un cycle aromatique éventuellement substitué ou un hétérocycle comprenant un ou deux atomes d'azote, éventuellement substitué,

. X représente un atome d'hydrogène ou un groupement alkyle linéaire comprenant 1 à 6 atomes de carbone ou un noyau phényle éventuellement substitué,

. Y représente une liaison ou un groupement alkyle, linéaire ou ramifié, comprenant de 1 à 10 atomes de carbone et

. Z représente un atome d'hydrogène,

- ou un groupement alkyle linéaire ou ramifié, contenant 1 à 11 atomes de carbone, et pouvant déterminer un cycle saturé constitué de 4 à 7 atomes de carbone,
- ou un groupement aralkyle, comprenant 7 à 12 atomes de carbone,
- ou un enchainement aliphatique de 1 à 4 groupements

méthylène, lié à un hétérocycle à 5 ou 6 chainons, l'hétéroatome étant choisi parmi l'azote, l'oxygène, le soufre.

- ou un groupement hétéroalkyle, comprenant de 1 à 10 atomes de carbone, l'hétéroatome étant choisi parmi l'azote, l'oxygène, le soufre.

Les dérivés selon l'invention sont des agents antibactériens remarquables à large spectre possédant une activité diurétique et ils présentent de plus des activités bronchodilatatrices intéressantes. Ils antagonisent l'histamine, l'acétylcholine et la sérotonine et se révélent antidépresseurs in vivo.

La signification du cycle aromatique Q s'applique par exemple à un dérivé de l'acide oxolinique où Q est un méthylènedioxy-phényle ou bien à l'acide nalidixique, Q déterminant avec le groupement pyridone carboxylique un motif ethyl-1 oxo-4 dihydro-1,4 méthyl-7 naphtyridinyl-3.

Lorsque Q représente une piperazinyl-2 fluoro-3 pyridine 5-6 condensée, le motif aromatique représente l'ENOXACINE. Lorsque Q signifie une piperazinyl-2 pyrimidine 5-6 condensée, on obtient alors le motif de l'acide PIPEMIDIQUE.

Lorsque Q représente un cycle phényle, celui-ci peut être substitué pour déterminer l'acide ethyl-1 oxo-4 fluoro-6 [(méthyl-4) piperazinyl-1]-7 dihydro-1,4 quinoline-3 carboxylique ou PEFLOXACINE.

Si le substituant-7 est une piperazine non substituée le motif aromatique décrit la NORFLOXACINE.

Si Q représente un cycle phényle et qu'il est substitué par un radical pyridinyl, le groupe aromatique représente alors la ROSOXACINE ou l'acide éthyl-1 oxo-4 (pyridyl-4)-7 dihydro-1,4 quinoléine-3 carboxylique.

La présente invention a également pour objet la préparation des composés répondant à la formule générale (I). Elle fait intervenir l'alkylation d'un sel alcalin ou

alcalino-terreux d'un acide de formule générale (II),

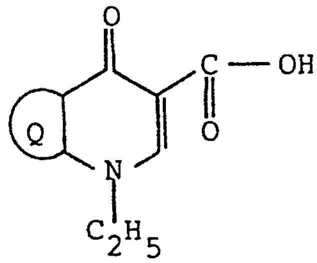

(II)

dans laquelle Q a la même signification que ci-dessus. L'agent alkylant est choisi parmi les halogénures d'alkyle répondant à la formule générale (III),

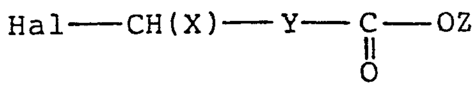

(III)

dans laquelle Hal représente un atome de chlore ou de brome, plus avantageusement un atome de brome et X, Y, Z ont les significations précédemment définies.

Les composés répondant à la formule (III) sont préparés par acylation d'un alcool Z-OH, Z étant différent de l'hydrogène ou d'un groupement ter-butyle, par le bromure de l'acide monobromoacétique ou le chlorure de l'acide monochloroacétique. L'acylation est réalisée dans un solvant inerte, tels que des haloalkanes, des éthers, des cétones, seuls ou mélangés, plus précisément choisis parmi le chloroforme, le chlorure de méthylène, l'éther diéthylique, le tetrahydrofuranne, l'acétone. La réaction est généralement conduite en présence d'un accepteur d'acide, plus exactement la pyridine ou la triéthylamine, à une température comprise entre 20° et 110°, plus généralement à température ambiante.

Les composés répondant à la formule générale (III), dans lesquels Z est un groupement t. butyle, sont préparés selon une technique connue (Bull. Soc. Chim. Fr., (1974), 12, 2985-6), en catalyse acide par l'acide sulfurique concentré, en présence d'alcool t. butylique, à température ordinaire.

La réaction conduisant aux composés de formule générale (I), entre le sel alcalin ou alcalino-terreux du composé de formule générale (II) et le composé de formule générale (III), est réalisée à une température comprise entre 20 et 100°, plus avantageusement à température ordinaire, dans un solvant inerte. Le solvant est choisi parmi le diméthylsulfoxyde, l'hexaméthylphosphoretriamide, ou des amides tels que le N,N diméthylformamide ou le N,N diméthylacétamide, le plus souvent le N,N diméthylformamide. Les composés de formule générale (I), avec Q, X et Y ayant la même signification que précédemment, et où Z représente un atome d'hydrogène, sont aisément obtenus par hydrolyse acide, plus avantageusement avec l'acide trifluoroacétique, d'un dérivé de formule générale (I), où Z représente un groupement t. butyle. L'hydrolyse est effectuée à une température comprise entre 0 et 30°. Lorsque Z représente un groupement benzyle, ils peuvent également être obtenus par hydrogénolyse en présence de palladium sur charbon.

L'invention sera mieux comprise à l'aide du complément de description qui va suivre, qui se réfère à des exemples de préparation de produits conformes à l'invention, ainsi qu'à un compte-rendu d'expérimentation relatif à l'activité pharmacologique de produits conformes à l'invention. Il doit être bien entendu, toutefois, que ces exemples et compte-rendus sont donnés uniquement à titre d'illustration de l'objet de l'invention dont ils ne constituent en aucune manière une limitation.

Tous les produits obtenus ont été soumis à l'analyse par chromatographie sur couche mince et ne présentent qu'un seul spot. Les chromatographies sur couche mince (CCM) ont été réalisées sur plaque Kieselgel F 254 et développées, dans les systèmes suivants :

- A : toluène 10 ; formiate d'éthyle 10 ; acide formique 1

- B : chloroforme 30 ; méthanol 2
- C : n. butanol 8 ; acide acétique 1 ; eau 1

et révélées en lumière ultra-violette à 254 nm. Les résultats des analyses centésimales pratiquées sur tous les produits sont conformes aux formules théoriques calculées.

EXEMPLE I - [ETHYL-1 OXO-4 DIHYDRO-1,4 MÉTHYL-7 NAPHTYRIDINYL-3] CARBONYLOXY-4, BUTYRATE D'ETHYLE

A une suspension de 17,8 g (70 mM) du sel de sodium de l'acide [ethyl-1 oxo-4 dihydro-1,4 méthyl-7 naphtyridinyl-3] carboxylique, dans 450 ml de diméthylformamide, on ajoute en 15 minutes, à température ordinaire, une solution de 13,85 g (71 mM) de bromo-4 butyrate d'éthyle dans 50 ml de diméthylformamide. L'agitation est maintenue pendant 48 heures à température ordinaire, et le solvant éliminé sous pression réduite.

On reprend le résidu avec 150 ml de soude 0,2 N glacée, on extrait au chloroforme, puis lave la couche chloroformique à la soude 0,5 N, à l'eau, on sèche sur sulfate de sodium. Après évaporation du chloroforme, le résidu est trituré à l'éther éthylique, puis repris dans l'éther de pétrole à froid. On filtre, sèche et obtient 47 % d'un produit pur, Rf = 0,23 (A), Rf = 0,86 (B), pF = 89-90°.

Spectre de RMN, en solution dans $CDCl_3$, par rapport au TMS : 9,05 ppm, (s), N-CH=, 1 H ; 7,25 et 8,60 ppm,(m), H arom, 2 H ; 4,45 ppm, (q),$N\underline{CH_2}CH_3$, 3 H ; 4,15 ppm (m), $COO\underline{CH_2}-$, 4 H ; 2,70 ppm, (s), $C(\underline{CH_3})-N$, 3 H ; 2,25 et 2,60 ppm, (m), $-CH_2-\underline{CH_2}-\underline{CH_2}$ COO-, 4 H ; 1,30 et 1,55 ppm, (2t) $N-CH_2\underline{CH_3}$ et $COOCH_2-\underline{CH_3}$, 6 H.

EXEMPLE II - [ETHYL-1 OXO-4 MÉTHYLÈNEDIOXY-6,7 DIHYDRO-1,4 QUINOLINYL-3] CARBONYLOXY-4 BUTYRATE D'ETHYLE

A une suspension de 19,8 g (70 mM) du sel de sodium de l'acide [ethyl-1 oxo-4 méthylènedioxy-6,7 dihydro-1,4 quinolinyl-3] carboxylique dans 450 ml de diméthyl formamide, on ajoute, en 15 minutes, à température

ambiante, une solution de 13,85 g (71 mM) de bromo-4 butyrate d'éthyle dans 50 ml de diméthylformamide. On maintient l'agitation pendant 48 heures à température ordinaire, on élimine le solvant sous pression réduite. Le résidu d'évaporation est repris dans 150 ml de soude 0,2 N glacé, on extrait au chloroforme, on lave à la soude 0,5 N, à l'eau, on sèche sur sulfate de sodium. On évapore le chloroforme, reprend le résidu à l'éther éthylique, évapore et reprend à l'éther de pétrole, à température ordinaire, on filtre, sèche et obtient 48 % d'un produit pur, Rf = 0,08 (A), Rf = 0,62 (B), Rf = 0,48 (C), pF = 105-7°.

Spectre de RMN, en solution dans $CDCl_3$, par rapport au TMS : 8,30 ppm, (s), N-<u>CH</u>=C-, 1 H ; 6,85 et 7,35 ppm, (2s), H aromatique en ortho du méthylène dioxy, 2 H ; 6,10 ppm (s), O-$\underline{CH_2}$-O, 2 H ; 4,35 ppm, (m), COO-$\underline{CH_2}$ ; 4,15 ppm,(q),N-$\underline{CH_2}$ $CH_3$, 2 H ; 2,10 et 2,50 ppm, (q et t), -COO $CH_2$ $\underline{CH_2}$ $\underline{CH_2}$ COO-, 4 H ; 1,25 et 1,50 ppm, (2t), N $CH_2$ $\underline{CH_3}$ et COO $CH_2$ $\underline{CH_3}$, 6 H.

EXEMPLE III - [ETHYL-1 OXO-4 DIHYDRO-1,4 MÉTHYL-7 NAPHTYRIDINYL-3] CARBONYLOXY-2 BUTYRATE D'ETHYLE

On ajoute 13,85 g (71 mM) de bromo-2 butyrate d'éthyle à une suspension de 17,8 g (70 mM) du sel de sodium de l'acide [ethyl-1 oxo-4 dihydro-1,4 méthyl-7 naphtyridinyl-3] carboxylique, dans 500 ml de diméthylformamide. On assure une agitation pendant 48 heures, à température ambiante, puis on évapore à sec. Le résidu est repris par 150 ml de soude 0,2 N glacée, puis extrait au chloroforme. La couche chloroformique est lavée à la soude 0,5 N, à l'eau, puis séchée sur sulfate de sodium. Après évaporation, le résidu est trituré dans l'éther éthylique, l'éther est évaporé, le résidu empaté dans l'éther de pétrole. Après filtration et séchage, on obtient 69 % d'un produit pur ; Rf = 0,40 (A) ; Rf = 0,82 (B) ; Rf = 0,78 (C) ; pF = 118-9°.

Spectre de RMN, en solution dans $CDCl_3$, par rapport au TMS : 9,05 ppm, (s), N-CH=, 1 H ; 7,25 et 8,60 ppm,(m), H arom, 2 H ; 5,15 ppm,(t), COOCH($CH_3$)COO, 1 H ; 4,40 ppm, (q), $NCH_2$ $CH_3$, 2 H ; 4,20 ppm, (q), $COOCH_2$ $CH_3$, 2 H ; 2,65 ppm (s), C($CH_3$)-N, 3 H ; 2,05 ppm, (m), -CH-$CH_2$ $CH_3$, 2 H ; 1,15 à 1,60 ppm, (m) -$CH_2$-$CH_3$, 9H.

EXEMPLE IV - [ETHYL-1 OXO-4 MÉTHYLÈNEDIOXY-6,7 DIHYDRO-1,4 QUINOLINYL-3] CARBONYLOXY-2 BUTYRATE D'ETHYLE

On ajoute 15,8 g (81 mM) de bromo-2 butyrate d'éthyle à une suspension de 22,65 g (80 mM) du sel de sodium de l'acide [ethyl-1 oxo-4 dihydro-1,4 méthyl-7 naphtyridinyl-3] carboxylique dans 550 ml de diméthylformamide. On maintient l'agitation à température ordinaire, pendant 48 heures, puis on évapore à sec. Le résidu est repris dans 150 ml de soude 0,2 N glacée, extrait au chloroforme, lavé à la soude 0,5 N, puis à l'eau, on sèche alors sur sulfate de sodium, et on évapore. Le résidu est empaté dans l'éther de pétrole, à froid, filtré, séché. On obtient ainsi 45 % d'un produit pur ; Rf = 0,10 (A) ; Rf = 0,63 (B) ; Rf = 0,67 (C) ; pF = 161-3°.

Spectre de RMN, en solution dans $CDCl_3$, par rapport au TMS : 8,35 ppm, (s), NCH=C-, 1 H ; 6,85 et 7,70 ppm, (2s), H arom, 2 H ; 6,10 ppm, (s), O-$CH_2$-O, 2H ; 5,15 ppm, (t), COOCH-, 1 H ; 4,20 ppm, (m), N-$CH_2$ et $COOCH_2$-, 4 H ; 2,0 ppm, (m), -CH-$CH_2$ $CH_3$, 2 H ; 1,05 à 1,50 ppm, (m), -$CH_2$-$CH_3$, 9 H.

EXEMPLE V - [ETHYL-1 OXO-4 DIHYDRO-1,4 MÉTHYL-7 NAPHTYRIDINYL-3] CARBONYLOXY-5 VALERATE D'ETHYLE

On ajoute 11,7 g (56 mM) de bromo-5 valérate d'éthyle à une suspension de 14 g (55 mM) du sel de sodium de l'acide [ethyl-1 oxo-4 dihydro-1,4 méthyl-7 naphtyridinyl-3] carboxylique, dans 400 ml de diméthylformamide. On agite à température ordinaire pendant 48 heures, puis on évapore à sec. Le résidu est repris dans 120 ml de soude 0,2 N glacée, et extrait au chloroforme. On lave la

couche chloroformique à la soude 0,5 N, à l'eau, on sèche et évapore. Le résidu pateux est empaté dans l'éther de pétrole, à froid. On filtre, sèche et obtient 25 % d'un produit pur, Rf = 0,78 (B), Rf = 0,75 (C), pF = 114-5°. Spectre de RMN, en solution dans $CDCl_3$, par rapport au TMS : 9,10 ppm, (s), N-CH=, 1 H ; 7,25 et 8,60 ppm, (m), H arom, 2 H ; 4,10 à 4,55 ppm, (m), $NCH_2$ $CH_3$, 2 $COOCH_2$, 6 H ; 2,65 ppm, (s) =$C(CH_3)$-N, 3 H ; 2,40 ppm, (t), $CH_2COO-$, 2 H ; 1,85 ppm, (m), $-CH_2$ $CH_2-$, 4 H ; 1,30 à 1,55 ppm, (2t), N $CH_2$ $CH_3$ et COO $CH_2$ $CH_3$, 6 H.

EXEMPLE VI - [ETHYL-1 OXO-4 MÉTHYLÈNEDIOXY-6,7 DIHYDRO -1,4 QUINOLINYL-3] CARBONYLOXY-5 VALERATE D'ETHYLE

On ajoute 11,7 g (56 mM) de bromo-5 valérate d'éthyle, à une suspension de 15,6 g (55 mM), du sel de sodium de l'acide [ethyl-1 oxo-4 méthylènedioxy-6,7 dihydro-1,4 quinolinyl-3] carboxylique dans 370 ml de diméthylformamide. On agite à température ordinaire pendant 48 heures, évapore à sec. Le résidu est repris dans 120 ml de soude 0,2 N glacée, extrait au chloroforme. On lave la couche chloroformique à la soude 0,5 N, à l'eau, sèche et évapore. Le résidu huileux est recouvert d'éther de pétrole pendant 15 heures. On filtre, sèche et obtient 33 % d'un produit pur, Rf = 0,57 (B) ; Rf = 0,56 (C), pF = 139-40°.
Spectre de RMN, en solution dans $CDCl_3$, par rapport au TMS : 8,30 ppm, (s), N-CH=, 1 H ; 6,85 et 7,80 ppm, (2s), H arom, 2 H ; 6,10 ppm, (s), $OCH_2O$, 2 H ; 4,10 et 4,20 ppm, (m), N-$CH_2$ et $COOCH_2-$, 4 H ; 2,40 ppm, (t), $CH_2$ COO, 2 H ; 1,80 ppm, (m), $-CH_2-CH_2-$, 4 H ; 1,20 à 1,55 ppm, (2t), N-$CH_2$ $CH_3$ et $COOCH_2$ $CH_3$, 6 H.

EXEMPLE VII - [ETHYL-1 OXO-4 DIHYDRO-1,4 MÉTHYL-7 NAPHTYRIDINYL-3] CARBONYLOXYACETATE DE n-HEPTYLE

1) Bromoacétate d'heptyle :

On coule 101 g (0,5 M) du bromure de l'acide bromoacétique sur une solution de 58 g (0,5 M) d'heptanol, 50,7 g (0,5 M) de triéthylamine, dans 600 ml d'éther anhydre, maintenu à 10°. On agite à température ordinaire pendant 15 heures, filtre, lave la couche éthérée à l'eau, sèche et distille. On obtient 56 % d'un produit pur, EB = 85° (0,25 mm).

2) On ajoute 11,9 g (50 mM) du bromoacétate de n-heptyle à une suspension de 13,2 g (52 mM) du sel de sodium de l'acide [ethyl-1 oxo-4 dihydro-1,4 méthyl-7 naphtyridinyl-3] carboxylique, dans 400 ml de diméthylformamide. On agite à température ordinaire pendant 48 heures, on évapore à sec, on reprend le résidu dans 120 ml de soude 0,2 N glacée. On extrait au chloroforme, lave à la soude 0,5 N, à l'eau, sèche et évapore. Le résidu huileux est recouvert d'éther de pétrole et abandonné à température ordinaire pendant 15 heures. On filtre et obtient 90 % d'un produit pur, Rf = 0,46 (A) ; Rf = 0,85 (B) ; pF = 59°.

Spectre de RMN en solution dans $CDCl_3$ par rapport au TMS : 8,75 ppm, (s), N=<u>CH</u>-,1 H ; 7,20 et 8,55 ppm (m), H arom, 2 H; 4,85 ppm, (s), $-COO\underline{CH_2}COO-$, 2 H ; 4,10 à 4,60 ppm, (q) et (t) $N-\underline{CH_2}-$ et COO $\underline{CH_2}$ $CH_2$,4 H ; 2,65 ppm, (s), $-C(\underline{CH_3})-N$, 3 H ; 1,20 à 1,70 ppm, (m), $-(\underline{CH_2})_5-$, $N-CH_2$ $\underline{CH_3}$, 13 H ; 0,85 ppm, (t), $-(CH_2)_6$ $\underline{CH_3}$, 3 H.

EXEMPLE VIII - [ETHYL-1 OXO-4 MÉTHYLÈNEDIOXY-6,7 DIHYDRO-1,4 QUINOLINYL-3] CARBONYLOXYACETATE DE n-HEPTYLE

On ajoute 11,9 g (50 mM) de bromoacétate d'heptyle, préparé selon la technique précédemment explicitée (ex VII-1), à une suspension de 14,7 g (52 mM) du sel de sodium de l'acide [ethyl-1 oxo-4 méthylènedioxy-6,7 dihydro-1,4 quinolinyl-3] carboxylique, dans 400 ml de diméthylformamide. On agite à température ordinaire pendant 48 heures, évapore la solution obtenue, et reprend

le résidu dans 100 ml de soude 0,2 N glacée ; on extrait au chloroforme et lave à la soude 0,5 N, à l'eau, sèche et évapore. Le résidu huileux est repris dans 200 ml d'éther de pétrole, abandonné à température ordinaire pendant 15 heures. On filtre et obtient 92 % d'un produit pur, Rf = 0,13 (A) ; Rf = 0,67 (B) ; pF = 97-8°.

Spectre de RMN, en solution dans $CDCl_3$, par rapport au TMS : 8,40 ppm, (s), N-CH=, 1 H ; 6,85 et 7,70 ppm, (2s), H arom, 2H ; 6,05 ppm, (s), $-O-\underline{CH_2}-O-$, 2 H ; 4,85 ppm, (s), $COO\underline{CH_2}$ COO-, 2 H ; 4,15 ppm, (m), $N-\underline{CH_2}-$ et $COO-\underline{CH_2}-$, 4 H ; 1,20 à 1,70 ppm, $N-CH_2$ $\underline{CH_3}$, $-(\underline{CH_2})_5-$, 13 H ; 0,90 ppm, (t), $-(CH_2)_6$ $\underline{CH_3}$, 3H.

EXEMPLE IX - [ETHYL-1 OXO-4 DIHYDRO-1,4 MÉTHYL-7 NAPHTYRIDINYL-3] CARBONYLOXYACETATE DE n-NONYLE

1) Bromoacétate de nonyle :

On coule 101 g (0,5 M) de bromure de l'acide bromoacétique sur une solution de 72 g (0,5 M) de nonanol, 50,7 g (0,5 M) de triéthylamine, dans 600 ml d'éther éthylique anhydre, maintenu à 10°. On agite à température ordinaire pendant 15 heures, filtre, évapore le solvant et distille sous pression réduite. On obtient 52 % d'un produit pur, EB = 95° (0,25 mm).

2) On ajoute 13,25 g (50 mM) du bromoacétate de nonyle à une suspension de 13,2 g (52 mM) du sel de sodium de l'acide éthyl-1, dihydro-1,4, méthyl-7, oxo-4, naphtyridinyl-3 carboxylique, dans 400 ml de diméthylformamide. On agite à température ordinaire pendant 48 heures, et on évapore à sec la solution obtenue. On reprend le résidu dans 150 ml de soude 0,2 N glacée, extrait au chloroforme.

On lave la couche chloroformique à la soude 0,5 N, à l'eau, on sèche et évapore. Le résidu huileux est recouvert d'éther de pétrole pendant 15 heures. On filtre et obtient 85 % d'un produit pur, Rf = 0,54 (A) ;

Rf = 0,86 (B) ; pF = 56-7°.
Spectre de RMN, en solution dans $CDCl_3$, par rapport au TMS : 8,70 ppm, (s), N=<u>CH</u>-, 1 H ;7,20 et 8,60 ppm,(m), H arom, 2 H ; 4,85 ppm, (s),-COO<u>CH</u>$_2$COO-, 2 H ; 4,10 à 4,65 ppm, (q) et (t), N-<u>CH</u>$_2$-$CH_3$ et COO <u>CH</u>$_2$$CH_2$, 4 H ; 2,65 ppm, (s), -C(<u>CH</u>$_3$)-N, 3 H ; 1,20 à 1,70 ppm, (m), -(<u>CH</u>$_2$)$_7$ $CH_3$ et N-$CH_2$ <u>CH</u>$_3$, 17 H ; 0,85 ppm, (t), $(CH_2)_7$ <u>CH</u>$_3$, 3 H.

<u>EXEMPLE X - [ETHYL-1 OXO-4 MÉTHYLÈNEDIOXY-6,7 DIHYDRO-1,4 QUINOLINYL-3] CARBONYLOXYACETATE DE n-NONYLE</u>

On ajoute 13,25 g (50 mM) de bromoacétate de n-nonyle, préparé selon la technique explicitée dans l'exemple IX-1), à une suspension de 14,7 g (52 mM) du sel de sodium de l'acide [ethyl-1 oxo-4 méthylènedioxy-6,7 dihydro-1,4 quinolinyl-3] carboxylique dans 400 ml de diméthylformamide. On agite à température ordinaire pendant 48 heures, et on évapore à sec la solution obtenue. On reprend le résidu dans 150 ml de soude 0,2 N glacée, extrait au chloroforme. On lave la couche chloroformique à la soude 0,5 N, à l'eau, on sèche et évapore. Le résidu huileux est recouvert d'éther de pétrole pendant 2 heures, puis on filtre. On obtient 88 % d'un produit pur, Rf = 0,15 (A) ; Rf = 0,68 (B) ; pF = 82°.
Spectre de RMN, en solution dans $CDCl_3$, par rapport au TMS : 8,35 ppm, (s), N-<u>CH</u>=,1 H ; 6,85 et 7,70 ppm, (2s), H arom, 2 H ; 6,10 ppm, (s) -O-<u>CH</u>$_2$-O-, 2 H ; 4,85 ppm, (s), COO-<u>CH</u>$_2$-COO, 2 H ; 4,05 à 4,30 ppm, (m), N-<u>CH</u>$_2$ $CH_3$ et COO-<u>CH</u>$_2$-$CH_2$-, 4 H ; 1,15 à 1,70 ppm, (m), $NCH_2$ <u>CH</u>$_3$, -$(CH_2)_7$-, 17 H ; 0,85 ppm, (t), -(<u>CH</u>$_2$)$_7$-<u>CH</u>$_3$, 3 H.

<u>EXEMPLE XI - [ETHYL-1 OXO-4 DIHYDRO-1,4 MÉTHYL-7 NAPHTYRIDINYL-3] CARBONYLOXYACETATE D'ETHYL-2 BUTYLE</u>

1) Bromoacétate d'éthyl-2 butyle :

On coule 101 g (0,5 M) du bromure de l'acide bromoacétique sur une solution de 51,1 g (0,5 M) d'alcool

correspondant, 51,6 g (0,51 M) de triéthylamine, dans 600 ml d'éther anhydre, maintenu à 10°. On agite à température ordinaire pendant 15 heures, filtre, évapore le solvant et distille sous pression réduite. On obtient 57 % d'un produit pur, EB = 50° (0,15 mm).

2) On ajoute 12,3 g (55 mM) du bromure précédent à une suspension de 14,23 g (56 mM) du sel de sodium de l'acide [ethyl-1 oxo-4 dihydro-1,4 méthyl-7 naphtyridinyl-3] carboxylique dans 400 ml de diméthylformamide. On agite à température ordinaire pendant 48 heures et on évapore à sec la solution obtenue. On reprend le résidu dans 150 ml de soude 0,2 N glacée, extrait au chloroforme. On lave la couche chloroformique à la soude 0,5 N, à l'eau, on sèche et évapore. Le résidu huileux est recouvert d'éther de pétrole, puis filtré ; On obtient ainsi 90 % d'un produit pur, Rf = 0,45 (A) ; Rf = 0,77 (B); pF = 82°.

Spectre de RMN, en solution dans $CDCl_3$, par rapport au TMS : 8,70 ppm (s), N=<u>CH</u>, 1 H ; 7,25 et 8,55 ppm, (m), H arom, 2 H ; 4,85 ppm, (s), -COO<u>$CH_2$</u>COO-, 2 H ; 4,10 et 4,50 ppm, (q) et (d), N<u>$CH_2$</u> $CH_3$ et COO <u>$CH_2$</u> CH-, 4 H ; 2,65 ppm, (s), =C(<u>$CH_3$</u>)-N, 3 H ; 1,15 à 1,65 ppm, (m), N-$CH_2$ <u>$CH_3$</u> et <u>CH</u>(<u>$CH_2$</u> $CH_3$) <u>$CH_2$</u> $CH_3$, 8 H; 0,85 ppm, (t), -$CH_2$<u>$CH_3$</u>, 6 H.

<u>EXEMPLE XII - [ETHYL-1 OXO-4 MÉTHYLÈNEDIOXY-6,7 DIHYDRO-1,4 QUINOLINYL-3] CARBONYLOXYACETATE D'ETHYL-2 BUTYLE</u>

On ajoute 12,3 g (55 mM) du bromure explicité dans l'exemple XI-1) à une suspension de 15,85 g (56 mM) du sel de sodium de l'acide [ethyl-1 oxo-4 méthylènedioxy-6,7 dihydro-1,4 quinolinyl-3] carboxylique, dans 400 ml de diméthylformamide. On agite à température ordinaire pendant 48 heures et on évapore à sec la solution obtenue. On reprend le résidu dans 150 ml de soude 0,2 N glacée, extrait au chloroforme.

La couche chloroformique est lavée à la soude 0,5 N, à l'eau, séchée et concentrée. Le résidu huileux est recouvert par 150 ml d'éther de pétrole pendant 15 heures. Après filtration et séchage, on obtient 93 % d'un produit pur, Rf = 0,2 (A) ; Rf = 0,61 (B) ; pF = 102-4°.

Spectre de RMN, en solution dans $CDCl_3$, par rapport au TMS : 8,35 ppm, (s), N-$\underline{CH}$=, 1 H ; 6,80 et 7,65 ppm, (2s), H arom, 2 H ; 6,10 ppm,(s), -O $\underline{CH_2}$O-, 2 H ; 4,85 ppm, (s), -COO$\underline{CH_2}$COO-, 2 H ; 4,05 à 4,35 ppm, (m), N-$\underline{CH_2}CH_3$ et COO$\underline{CH_2}$CH-, 4 H ; 1,20 à 1,60 ppm, (m), N-$CH_2\underline{CH_3}$ et $\underline{CH}(\underline{CH_2}CH_3)\underline{CH_2}CH_3$, 8 H ; 0,75 à 1 ppm, (m), -$CH_2$ $\underline{CH_3}$, 6 H.

EXEMPLE XIII - [ETHYL-1 OXO-4 DIHYDRO-1,4 MÉTHYL-7 NAPHTYRIDINYL-3] CARBONYLOXYACETATE D'ETHYL-2 HEXYLE

1) Bromoacétate d'éthyl-2 hexyle :

On coule 111 g (0,55 M) du bromure de l'acide bromoacétique sur une solution de 71,6 g (0,55 M) d'éthyl-2 hexanol, 56,7 g (0,56 M) de triéthylamine dans 700 ml d'éther anhydre, maintenu à 10°. On agite à température ordinaire pendant 15 heures, filtre, évapore le solvant et distille sous pression réduite. On obtient 68 % d'un produit pur, EB = 65-7° (0,03 mn).

On ajoute 13,8 g (55 mM) du bromure précédent à une suspension de 14,25 g (56 mM) du sel de sodium de l'acide [ethyl-1 oxo-4 dihydro-1,4 méthyl-7 naphtyridinyl-3] carboxylique dans 400 ml de diméthylformamide. On agite à température ordinaire pendant 48 heures et évapore à sec la solution obtenue. On reprend le résidu dans 150 ml de soude 0,2 N glacée, extrait au chloroforme, lave la couche chloroformique à la soude 0,5 N, à l'eau, sèche et évapore. Le résidu est recouvert d'éther de pétrole. Après filtration, séchage, on obtient 87 % d'un produit pur, Rf = 0,45 (A) ; Rf = 0,80 (B) ; pF = 65-6°.

Spectre de RMN, en solution dans $CDCl_3$, par rapport au TMS : 8,65 ppm, (s), N-$\underline{CH}$-, 1 H ; 7,20 et 8,55 ppm,

(m), H arom, 2 H ; 4,85 ppm, (s), $-COO\underline{CH_2}COO-$, 2 H ; 4,10 et 4,50 ppm, (q) et (d), $N\underline{CH_2}CH_3$ et $COO\underline{CH_2}CH-$, 4 H ; 2,65 ppm, (s), $=C(\underline{CH_3})-N$, 3 H ; 1,15 à 1,65 ppm, (m), $N-CH_2\ \underline{CH_3}$ et $\underline{CH}(\underline{CH_2}CH_3)\underline{CH_2}\ \underline{CH_2}\ \underline{CH_2}\ CH_3$, 12 H ; 0,90 ppm, (t), $-CH_2\ \underline{CH_3}$, 6 H.

EXEMPLE XIV - [ETHYL-1 OXO-4 MÉTHYLÈNEDIOXY-6,7 DIHYDRO-1,4 QUINOLINYL-3] CARBONYLOXYACETATE D'ETHYL-2 HEXYLE

On ajoute 13,8 g (55 mM) du bromure explicité dans l'exemple XIII-1) à une suspension de 15,85 g (56 mM) du sel de sodium de l'acide [ethyl-1 oxo-4 méthylènedioxy-6,7 dihydro-1,4 quinolinyl-3] carboxylique dans 400 ml de diméthylformamide. On agite à température ordinaire pendant 48 heures et évapore à sec la solution obtenue. Le résidu est repris dans 150 ml de soude 0,2 N glacée, extrait au chloroforme. La couche chloroformique est lavée à la soude 0,5 N, à l'eau, séchée, évaporée, le résidu recouvert d'éther de pétrole pendant 15 heures. Après filtration et séchage, on obtient 92 % d'un produit pur, Rf = 0,17 (A) ; Rf = 0,60 (B) ; pF = 71-3°.

Spectre de RMN, en solution dans $CDCl_3$, par rapport au TMS : 8,35 ppm,(s), $N-\underline{CH}$, 1 H ; 6,80 et 7,65 ppm, (2s), H arom, 2 H ; 6,10 ppm, (s), $-O\underline{CH_2}O-$, 2 H ; 4,85 ppm, (s), $COO\ \underline{CH_2}\ COO$, 2 H ; 4 à 4,30 ppm, (m), $N\underline{CH_2}\ CH_3$ et $COO\ \underline{CH_2}\ CH-$, 4 H ; 1,05 à 1,60 ppm, (m), $N-CH_2\ \underline{CH_3}$ et $\underline{CH}(\underline{CH_2}\ CH_3)\ \underline{CH_2}\ \underline{CH_2}\ \underline{CH_2}\ CH_3$, 12 H ; 0,75 à 1 ppm, $-CH_2\ \underline{CH_3}$, 6 H.

EXEMPLE XV - [ETHYL-1 OXO-4 DIHYDRO-1,4 MÉTHYL-7 NAPHTYRIDINYL-3] CARBONYLOXYACETATE DE CYCLOHEXYLMETHYLE

1) Bromoacétate de cyclohexylméthyle

On coule 100,9 g (0,5 M) du bromure de l'acide bromoacétique sur une solution de 57,1 g (0,5 M) de cyclohexylméthanol, 51,6 g (0,51 M) de triéthylamine, dans 600 ml d'éther anhydre, à 10°. On agite à température ordinaire pendant 15 heures, filtre, évapore le solvant et

distille sous pression réduite. On obtient 60 % d'un produit pur, EB = 60 °C (0,25 mm).

2) On ajoute 12,9 g (55 mM) du bromure précédent à une suspension de 14,23 g (56 mM) du sel de sodium de l'acide [ethyl-1 oxo-4 dihydro-1,4 méthyl-7 naphtyridinyl-3] carboxylique, dans 400 ml de diméthylformamide. On agite à température ordinaire pendant 48 heures et on évapore à sec la solution obtenue. Le résidu est repris dans 150 ml de soude 0,2 N glacée, extrait au chloroforme. La couche chloroformique est lavée à la soude 0,5 N, à l'eau, séchée, concentrée. Le résidu huileux est recouvert d'éther de pétrole. Après filtration et séchage, on obtient 92 % d'un produit pur, Rf = 0,40 (A) ; Rf = 0,73 (B) ; pF = 130-2°.

Spectre de RMN, en solution dans $CDCl_3$, par rapport au TMS : 8,70 ppm, (s) N-<u>CH</u>, 1 H ; 7,20 et 8,55 ppm, (m), H arom, 2 H ; 4,85 ppm, (s), -COO <u>$CH_2$</u>COO-, 2 H ; 4,50 ppm, (q), N-<u>$CH_2$</u>$CH_3$, 2 H ; 4,0 ppm (d), COO<u>$CH_2$</u>CH-, 2 H ; 2,65 ppm, (s), =C(<u>$CH_3$</u>)-N, 3 H ; 1,15 à 1,85 ppm, -N $CH_2$ <u>$CH_3$</u> et -$CH_2$ <u>$C_6H_{11}$</u>, 14 H.

<u>EXEMPLE XVI - [ETHYL-1 OXO-4 MÉTHYLÈNEDIOXY-6,7 DIHYDRO-1,4 QUINOLINYL-3] CARBONYLOXYACETATE DE CYCLOHEXYLMETHYLE</u>

On ajoute 12,9 g (55 mM) du bromure, explicité dans l'exemple XV-1), à une suspension de 15,85 g (56 mM) du sel de sodium de l'acide [ethyl-1 oxo-4 méthylènedioxy-6,7 dihydro-1,4 quinolinyl-3] carboxylique, dans 400 ml de diméthylformamide.

On agite à température ordinaire pendant 48 heures et évapore à sec la solution obtenue. Le résidu est repris dans 150 ml de soude 0,2 N glacée, extrait au chloroforme. La couche chloroformique est lavée à la soude 0,5 N, à l'eau, séchée, évaporée, et le résidu recouvert d'éther de pétrole.

Après filtration et séchage, on obtient 92 % d'un

produit pur, Rf = 0,23 (A) ; Rf = 0,61 (B) ; pF = 119-20°.

Spectre de RMN, en solution dans $CDCl_3$, par rapport au TMS : 8,35 ppm, (s), N-CH, 1 H ; 6,80 et 7,65 ppm, (2s), H arom, 2 H ; 6,10 ppm, (s), $-OCH_2O-$, 2 H ; 4,85 ppm, (s), $COOCH_2COO-$, 2 H ; 3,95 à 4,30 ppm, (m), $N-CH_2$ $CH_3$ et COO $CH_2$ CH-, 4 H ; 0,90 à 1,85 ppm, $NCH_2$ $CH_3$ et $CH_2-C_6H_{11}$, 14 H.

EXEMPLE XVII - [ETHYL-1 OXO-4 DIHYDRO-1,4 MÉTHYL-7 NAPHTYRIDINYL-3] CARBONYLOXYACETATE DE TER-BUTYLE

On ajoute 13,5 g (69 mM) de bromo-2 acétate de t. butyle à une suspension de 17,8 g (70 mM) du sel de sodium de l'acide [ethyl-1 oxo-4 dihydro-1,4 méthyl-7 naphtyridinyl-3] carboxylique en suspension dans 500 ml de diméthylformamide. On agite à température ordinaire pendant 48 heures, on évapore à sec la solution obtenue. Le résidu est repris dans l'eau, on extrait au chloroforme, on lave la couche chloroformique à la soude 0,5 N glacée, à l'eau. Après séchage, on évapore et reprend pendant quelques heures à l'éther de pétrole. On filtre, sèche et obtient 88 % d'un produit pur, Rf = 0,79 (B) ; Rf = 0,85 (C) ; pF = 151-2°.

Spectre de RMN, en solution dans $CDCl_3$, par rapport au TMS : 8,70 ppm, (s), N=CH, 1 H ; 7,25 et 8,55 ppm, (m), H arom, 2 H ; 4,75 ppm, (s), $-COOCH_2COO-$, 2 H ; 4,45 ppm, (q), N $CH_2$ $CH_3$, 2 H ; 2,65 ppm, (s) $=C(CH_3)-N-$, 3 H ; 1,45 à 1,65 ppm, (s) et (t), N $CH_2CH_3$ et $-C(CH_3)_3$, 12 H.

EXEMPLE XVIII - [ETHYL-1 OXO-4 MÉTHYLÈNEDIOXY-6,7 DIHYDRO-1,4 QUINOLINYL-3] CARBONYLOXYACETATE DE TER-BUTYLE

On coule 13,4 g (69 mM) de bromoacétate de t-butyle sur une suspension de 19,8 g (70 mM) du sel de sodium de l'acide [ethyl-1 oxo-4 méthylènedioxy-6,7 dihydro-1,4 quinolinyl-3] carboxylique, dans 500 ml de diméthylformamide. On agite à température ordinaire pendant 48 heures, et évapore à sec la solution obtenue. On reprend le résidu avec 200 ml d'eau glacée, extrait au chloroforme.

La couche chloroformique est lavée à la soude 0,5 N, à l'eau, séchée et évaporée. Le résidu huileux cristallise à chaud dans l'éther de pétrole. Après filtration, séchage, on obtient 91 % d'un produit pur, Rf = 0,11 (A) ; Rf = 0,59 (B) ; pF = 176°.

Spectre de RMN, en solution dans $CDCl_3$, par rapport au TMS : 8,35 ppm, (s), -N-<u>CH</u>=, 1 H ; 6,80 et 7,65 ppm, (2s), H arom, 2 H ; 6,10 ppm, (s), -O-<u>CH</u>$_2$-O-, 2 H ; 4,70 ppm, (s), -COO-<u>CH</u>$_2$-COO-, 2 H ; 4,15 ppm, (q), N<u>CH</u>$_2$ $CH_3$, 2 H ; 1,45 ppm, (s), -C(<u>CH</u>$_3$)$_3$, 9 H ; 1,15 à 1,35 ppm, (t), N-$CH_2$<u>CH</u>$_3$, 3 H.

<u>EXEMPLE XIX - [ETHYL-1 OXO-4 MÉTHYLÈNEDIOXY-6,7 DIHYDRO-1,4 QUINOLINYL-3] CARBONYLOXYACETATE DE BENZYLE</u>

On coule 14 g (61 mM) de bromo-2 acétate de benzyle sur une suspension de 17 g (60 mM) du sel de sodium de l'acide [ethyl-1 oxo-4 méthylènedioxy-6,7 dihydro-1,4 quinolinyl-3] carboxylique, dans 400 ml de diméthylformamide. On agite à température ordinaire pendant 48 heures et on évapore à sec. Le résidu est repris avec 200 ml de soude 0,2 N, à froid, et extrait au chloroforme. On lave la couche chloroformique à la soude 0,5 N, à l'eau, sèche et évapore à sec. Le résidu solide est empaté dans 300 ml d'éther de pétrole. On filtre, sèche et obtient 91 % d'un produit pur, Rf = 0,78 (B), Rf = 0,64 (C), pF = 152-3°.

Spectre de RMN, en solution dans $CDCl_3$, par rapport au TMS : 8,35 ppm, (s), N-<u>CH</u>=, 1 H ; 6,80 et 7,70 ppm, (2s), H arom, 2 H ; 7,35 ppm, (s), $CH_2$-$C_6$<u>H</u>$_5$, 5 H ; 6,05 ppm, (s), -O-<u>CH</u>$_2$-O, 2 H ; 5,20 ppm, (s), <u>CH</u>$_2$-$C_6H_5$, 2 H ; 4,90 ppm, (s), -COO-<u>CH</u>$_2$-COO-, 2 H ; 4,10 ppm, (q), N-<u>CH</u>$_2$ $CH_3$, 2 H ; 1,50 ppm, (t), N $CH_2$ <u>CH</u>$_3$, 3 H.

EXEMPLE XX - [ETHYL-1 OXO-4 MÉTHYLÈNEDIOXY-6,7 DIHYDRO-1,4 QUINOLINYL-3] CARBONYLOXY-6 HEXANOATE DE T-BUTYLE

1) Bromo-6, hexanoate de t-butyle :

On coule 1,2 ml d'acide sulfurique concentré, goutte à goutte, sur un mélange de 48,8 g (0,25 M) d'acide 6-bromo hexanoïque et 5 ml d'alcool t-butylique. On porte le mélange à une température de 0° et coule rapidement 50,5 g (0,9 M) d'isobutylène préalablement liquéfié au freezer. On bouche hermétiquement et agite pendant 15 heures à température ordinaire. La solution obtenue est réfrigérée, traitée avec une solution saturée de bicarbonate de sodium jusqu'à un PH voisin de 7-8. On extrait à l'éther, lave à l'eau glacée, sèche et distille; On obtient 81 % d'un produit pur, EB = 126-8° (13 mm), Rf = 0,81 ($CHCl_3$).

2) On coule 12,6 g (50 mM) de bromo-6 hexanoate de t-butyle sur une suspension de 14,5 g (51 mM) du sel de sodium de l'acide [ethyl-1 oxo-4 méthylènedioxy-6,7 dihydro-1,4 quinolinyl-3] carboxylique dans 400 ml de diméthylformamide. On agite à température ordinaire pendant 48 heures, évapore à sec la solution, reprend le résidu avec 150 ml d'eau glacée, extrait au chloroforme. On lave la couche chloroformique à la soude 0,5 N, à l'eau, sèche et évapore. Le résidu huileux est empaté dans l'éther de pétrole, à froid, pendant quelques heures. On filtre, sèche et obtient 23 % d'un produit pur, Rf = 0,69 (B), Rf = 0,62 (C), pF = 81-3°.

Spectre de RMN, en solution dans le diméthylsulfoxyde $d_6$, par rapport au TMS : 8,60 ppm, (s), $-N-\underline{CH}=$, 1 H ; 7,45 et 7,60 ppm, (2s), H arom, 2 H ; 6,30 ppm, (s), $-O\underline{CH_2}O-$, 2 H; 4,25 et 4,45 ppm, (m), $N\underline{CH_2}CH_3$ et $COO\underline{CH_2}-(CH_2)_4-$, 4 H ; 2,30 ppm, (t), $-(CH_2)_4\ \underline{CH_2}COO-$, 2 H ; 1,50 à 1,70 ppm (m), $COOCH_2(\underline{CH_2})_3CH_2$, 6 H ; 1,20 à 1,45 ppm, (t) et (s), $-NCH_2\underline{CH_3}$, $C(\underline{CH_3})_3$, 12 H.

EXEMPLE XXI - ACIDE [ETHYL-1 OXO-4 DIHYDRO-1,4 MÉTHYL-7 NAPHTYRIDINYL-3] CARBONYLOXYACETIQUE

On additionne en une portion 13,85 g (40 mM) de l'acétate de t-butyle correspondant, préparé selon l'exemple XVII, à une solution de 140 ml d'acide trifluoroacétique, préalablement maintenue à 0°. On agite pendant 30 minutes à froid, puis pendant 2 heures à température ordinaire. On évapore à sec et dilue avec l'éther éthylique. On agite à froid pendant 30 mn, filtre et sèche. On obtient 96 % d'un produit pur, Rf = 0,05 (A), Rf = 0,23 (C), pF = 219-21°.

Spectre de RMN, en solution dans $CDCl_3$, par rapport au TMS : 8,70 ppm, (s), -N-CH=, 1 H ; 7,30 et 8,55 ppm, (m), H arom, 2 H ; 6,60 ppm, (m), -COOH, H échangeable eau, 1 H; 4,95 ppm, (s), $COO\underline{CH_2}COO$, 2 H ; 4,55 ppm, (q), $N\underline{CH_2}CH_3$, 2 H ; 2,70 ppm, (s), $=C(\underline{CH_3})-N-$, 3 H ; 1,50 ppm, (t), $NCH_2\underline{CH_3}$, 3 H.

EXEMPLE XXII - ACIDE [ETHYL-1 OXO-4 MÉTHYLÈNEDIOXY-6,7 DIHYDRO-1,4 QUINOLINYL-3] CARBONYLOXY ACETIQUE

On hydrogène à température ordinaire et pression atmosphérique 18,4 g (45 mM) de l'ester benzylique correspondant (explicité dans l'exemple XIX), dans 300 ml d'acide acétique, en présence de 2 g de palladium à 5 % sur C. Après filtration du catalyseur, à 80°, on évapore à sec et reprend le résidu avec de l'éther éthylique. On filtre, sèche et reprend le résidu solide à l'eau glacée, neutralise à la soude 2N et reprécipite avec l'acide chlorhydrique concentré, à froid. On obtient 92 % d'un produit pur, Rf = 0,12 (C), pF = 240-3°.

Spectre de RMN en solution dans NaOD 0,5 N : 8,60 ppm, (s), $-N-\underline{CH}=$, 1 H ; 6,95 et 7,30 ppm, (m), H arom, 2 H ; 6,25 ppm (s), $O\underline{CH_2}O$, 2 H ; 4,90 ppm, (s), $-COO\underline{CH_2}COO-$, 2 H ; 4,25 ppm, (m), $N\underline{CH_2}CH_3$, 2 H ; 1,50 ppm, (m), $NCH_2\underline{CH_3}$, 3 H.

EXEMPLE XXIII - ACIDE [ETHYL-1 OXO-4 MÉTHYLÈNEDIOXY-6,7 DIHYDRO-1,4 QUINOLINYL-3] CARBONYLOXY-6 HEXANOIQUE

On additionne 3,9 g (9 mM) de l'ester t-butylique correspondant (préparé selon l'exemple XX), à une solution de 25 ml d'acide trifluoroacétique, préalablement maintenue à 0°. On agite pendant 30 minutes à froid, 90 mn à température ordinaire, évapore à sec et recouvre avec 200 ml d'éther éthylique. On agite 30 mn à froid, filtre, sèche. On obtient 90 % d'un produit pur, Rf = 0,55 (C), pF = 207-9°.

EXEMPLE XXIV - [ETHYL-1 OXO-4 MÉTHYLÈNEDIOXY-6,7 DIHYDRO-1,4 QUINOLINYL-3] CARBONYLOXY-2 PROPIONATE DE BENZYLE

On coule 34 g (0,14 M) de bromo-2 propionate de benzyle, préparé selon la procédure détaillée dans l'exemple XI-1), sur une suspension de 39,6 g (0,14 M) du sel de sodium de l'acide [ethyl-1 oxo-4 méthylènedioxy-6,7 dihydro-1,4 quinolinyl-3] carboxylique dans 900 ml de diméthylformamide. On obtient, selon la technique explicitée pour le bromo-2 butyrate d'éthyle (exemple IV), 73 % d'un produit pur, Rf = 0,16 (A) ; Rf = 0,85 (B) ; pF = 129-30°.

Spectre de RMN, en solution dans $CDCl_3$, par rapport au TMS : 8,30 ppm, (s), -N-<u>CH</u>=, 1 H ; 6,80 et 7,60 ppm, (2s), H arom, 2 H ; 7,30 ppm, (s), $CH_2$ $\underline{C_6}$ $\underline{H_5}$, 5 H ; 6,05 ppm, (s), -O$\underline{CH_2}$O-, 2 H ; 5,15 à 5,45 ppm, (q), -<u>CH</u> $(CH_3)$-, 1 H; 5,20 ppm, (s), -$\underline{CH_2}$ $C_6$ $H_5$, 2 H ; 4,10 ppm, (q), N$\underline{CH_2}$ $CH_3$, 2 H ; 1,65 ppm, (d), -CH($\underline{CH_3}$)-, 3 H ; 1,35 ppm, (t), $NCH_2\underline{CH_3}$, 3 H.

EXEMPLE XXV - [ETHYL-1 OXO-4 MÉTHYLÈNEDIOXY-6,7 DIHYDRO-1,4 QUINOLINYL-3] CARBONYLOXY-2 BUTYRATE DE BENZYLE

On coule 34,7 g (0,135 M) de bromo-2 butyrate de benzyle, (préparé selon l'exemple XI-1) sur une suspension

de 38,2 g (0,135 M) du sel de sodium de l'acide [ethyl-1 oxo-4 méthylènedioxy-6,7 dihydro-1,4 quinolinyl-3] carboxylique, dans 900 ml de diméthylformamide. On obtient, selon la technique détaillée dans l'exemple IV, 40 % d'un produit pur, Rf = 0,10 (A) ; Rf = 0,60 (B) ; pF = 129-31°.

Spectre de RMN, en solution dans $CDCl_3$, par rapport au TMS : 8,50 ppm, (s), -N-<u>CH</u>=, 1 H ; 7,15 et 7,60 ppm, (2s), H arom, 2 H ; 7,30 ppm, (s), $CH_2$ <u>$C_6$</u> <u>$H_5$</u>, 5 H ; 6,10 ppm, (s), -O<u>$CH_2$</u>O-, 2 H ; 5 à 5,20 ppm, (s) et massif, -<u>CH</u>($C_2H_5$)- et <u>$CH_2$</u> $C_6H_5$, 3 H ; 4,10 à 4,45 ppm, (q), N<u>$CH_2$</u>$CH_3$, 2 H ; 1,95 ppm, (m), -CH(<u>$CH_2$</u>$CH_3$)-, 2 H ; 1,40 ppm, (t), N $CH_2$ <u>$CH_3$</u>, 3 H ; 1,05 ppm, (t),-CH($CH_2$<u>$CH_3$</u>), 3 H.

EXEMPLE XXVI - [ETHYL-1 OXO-4 MÉTHYLÈNEDIOXY-6,7 DIHYDRO-1,4 QUINOLINYL-3] CARBONYLOXY-2 HEXANOATE DE BENZYLE

On coule 31,4 g (0,11 M) de bromo-2 hexanoate de benzyle, préparé selon l'exemple XI-1), sur une suspension de 31,2 g (0,11 M) du sel de sodium de l'acide [ethyl-1 oxo-4 méthylènedioxy-6,7 dihydro-1,4 quinolinyl-3] carboxylique, dans 750 ml de diméthylformamide. On obtient, selon la technique détaillée dans l'exemple IV, 48 % d'un produit pur ; Rf = 0,21 (A) ; Rf = 0,71 (C) ; pF = 93°.

Spectre de RMN, en solution dans $CDCl_3$, par rapport au TMS : 8,30 ppm, (s), -N-<u>CH</u>=, 1 H ; 6,90 et 7,70 ppm, (2s), H arom, 2 H ; 7,35 ppm, (s), -$CH_2$ <u>$C_6$</u> <u>$H_5$</u>, 5 H ; 6,05 ppm, (s), -O<u>$CH_2$</u>O-, 2 H ; 5,10 à 5,40 ppm, (s) et massif, -<u>CH</u> ($C_4H_9$)- et <u>$CH_2$</u> $C_6$ $H_5$, 3 H ; 3,95 à 4,30 ppm, (q), N<u>$CH_2$</u> $CH_3$, 2 H ; 1,40 ppm, (t), N $CH_2$ <u>$CH_3$</u>, 3 H ; 0,90 à 2,05 ppm, (m), -CH ($C_4$ <u>$H_9$</u>)-, 9 H.

EXEMPLE XXVII - [ETHYL-1 OXO-4 MÉTHYLÈNEDIOXY-6,7 DIHYDRO-1,4 QUINOLINYL-3] CARBONYLOXY-2 PHENYL-2 ACETATE DE BENZYLE

On coule 33,6 g (0,11 M) de l'α bromo phénylacétate de benzyle, préparé selon l'exemple XI-1), sur une suspension de 31,2 g (0,11 M) du sel de sodium de l'acide [ethyl-1 oxo-4 méthylènedioxy-6,7 dihydro-1,4 quinolinyl-3] carboxylique dans 750 ml de diméthylformamide.
On obtient, selon la technique détaillée dans l'exemple IV, 89 % d'un produit pur, Rf = 0,27 (A) ; Rf = 0,82 (C), pF = 133-5°.
Spectre de RMN, en solution dans $CDCl_3$, par rapport au TMS : 8,50 ppm, (s), N-<u>CH</u>=, 1 H ; 6,95 à 7,65 ppm, (m), H arom, 12 H ; 6,10 ppm, (2s), $-O\underline{CH_2}O-$ et $-\underline{CH}\ (C_6\ H_5)-$, 3 H ; 5,10 ppm, (s), $-\underline{CH_2}\ C_6H_5$, 2 H ; 4,05 à 4,30 ppm, (q), $N\underline{CH_2}\ CH_3$, 2 H ; 1,35 ppm, (t), $-N\ CH_2\ \underline{CH_3}$, 3 H.

<u>EXEMPLE XXVIII - ACIDE [ETHYL-1 OXO-4 MÉTHYLÈNEDIOXY-6,7 DIHYDRO-1,4 QUINOLINYL-3] CARBONYLOXY-2 PROPIONIQUE</u>

A partir de l'ester benzylique correspondant (exemple XXIV), on obtient par hydrogénation catalytique selon la technique détaillée dans l'exemple XXII, 85 % d'un produit pur, Rf = 0,06 (B), Rf = 0,12 (C), pF = 219-21°.

<u>EXEMPLE XXIX - ACIDE [ETHYL-1 OXO-4 MÉTHYLÈNEDIOXY-6,7 DIHYDRO-1,4 QUINOLINYL-3] CARBONYLOXY-2 BUTYRIQUE</u>

A partir de l'ester benzylique correspondant (exemple XXV), on obtient, par hydrogénation catalytique, selon la technique détaillée dans l'exemple XXII, 90 % d'un produit pur, Rf = 0,08 (B), Rf = 0,21 (C) ; pF = 214-7°.

<u>EXEMPLE XXX - ACIDE [ETHYL-1 OXO-4 MÉTHYLÈNEDIOXY-6,7 DIHYDRO-1,4 QUINOLINYL-3] CARBONYLOXY-2 HEXANOIQUE</u>

A partir de l'ester benzylique correspondant (exemple XXVI), on obtient par hydrogénation catalytique selon la technique détaillée dans l'exemple XXII, 91 % d'un produit pur, Rf = 0,12 (B), Rf = 0,40 (C), pF = 131-4°.

Les antibactériens selon l'invention possèdent en

outre des propriétés pharmacologiques intéressantes. Ils se révèlent être des diurétiques, des relaxants musculaires, des bronchodilatateurs et des antidépresseurs.

Ainsi, dans le bronchospasme déclenché à l'acétylcholine chez l'animal, les activités bronchodilatatrices sont comparables voire supérieures à celle de la théophylline. Les bronchospasmes induits sur la trachée isolée de cobaye sont antagonisés par les dérivés selon l'invention et le propanolol ne diminue pas cet effet relaxant indiquant l'absence possible d'action $\beta_2$ agoniste.

Les dérivés selon l'invention antagonisent les différents agonistes : histamine, acétylcholine, sérotonine et chlorure de Baryum à des concentrations atteignant $2\text{-}10^{-7}$ Mole/ml. Ils inhibent l'activité inotrope induite par l'histamine.

A 30 mg/kg, ils se révèlent antidépresseurs chez la souris/selon Vermier - First Hahnemann - Symposium on Psychosomatic Medecines ed. Nodine et Mayer p. 683 - 1962. Aux mêmes doses, la plupart des produits selon l'invention présentent une activité diurétique chez le rat.

La demanderesse a également constaté que ces composés possèdent des activités antibactériennes remarquables vis-à-vis notamment des gram. négatifs et de groupes de bactéries résistants aux agents chimiothérapeutiques connus.

Les composés selon l'invention peuvent être utilisés comme médicaments sous la forme de préparations pharmaceutiques usuelles. Ces dernières peuvent être administrées par voie orale, parentérale, rectale, transdermique ou sous forme d'aérosol seuls ou avec les excipients adaptés à chaque forme galénique.

La posologie d'administration des composés selon l'invention varie selon les voies d'administration, l'âge,

le poids et l'état des patients et l'affection particulière à traiter. En général, on peut utiliser ces principes actifs à des doses journalières de 1 à 50 mg/kg et en particulier de 2 à 20 mg/kg.

REVENDICATIONS

1 - Antibactériens à action pharmacologique multiple caractérisés en ce qu'ils répondent à la formule générale suivante :

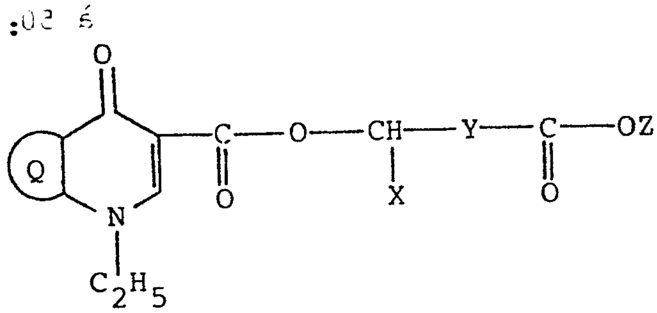

(I)

dans laquelle :

. Q représente un cycle aromatique éventuellement substitué ou un hétérocycle comprenant un ou deux atomes d'azote éventuellement substitué.

. X représente un atome d'hydrogène ou un groupement alkyle linéaire ou ramifié comprenant 1 à 6 atomes de carbone, un noyau phényl éventuellement substitué.

. Y représente une liaison ou un groupement alkyle linéaire ou ramifié comprenant 1 à 10 atomes de carbone.

. Z représente un atome d'hydrogène :

- ou un groupement alkyle linéaire ou ramifié contenant de 1 à 11 atomes de carbone et pouvant déterminer un cycle saturé constitué de 4 à 7 atomes de carbone.

- ou un groupement aralkyle comprenant de 7 à 12 atomes de carbone.

- ou un enchainement aliphatique de 1 à 4 méthylène lié à un hétérocycle à 5 ou 6 chainons, l'hétéroatome étant choisi parmi l'azote, le soufre, l'oxygène.

- ou un groupement hétéroalkyle comprenant de 1 à 10 atomes de carbone et l'hétéroatome étant choisi parmi l'azote, le soufre ou l'oxygène.

2 - Sels d'addition des composés selon la revendication 1 avec les acides ou les bases pharmaceutiquement acceptables.

3 - Les dérivés selon les revendications 1 et 2 où Q détermine avec la pyridone le dérivé ethyl-1 dihydro-1,4 méthylènedioxy-6,7 oxo-4 quinolyl-3.

4 - Les dérivés selon les revendications 1 et 2 où Q détermine avec la pyridone le dérivé ethyl-1 dihydro-1,4 méthyl-7 oxo-4 naphtyridinyl-3.

5 - Dérivé selon la revendication 1 caractérisé en ce qu'il est constitué par l'[ethyl-1 dihydro-1,4 méthyl-7 oxo-4 naphtyridinyl-3] carbonyloxy-4 butyrate d'éthyle.

6 - Dérivé selon la revendication 1 caractérisé en ce qu'il est constitué par l'[ethyl-1 oxo-4 méthylènedioxy-6,7 dihydro-1,4 quinolinyl-3] carbonyloxy-4 butyrate d'éthyle.

7 - Dérivé selon la revendication 1 caractérisé en ce qu'il est constitué par l'[ethyl-1 dihydro-1,4 méthyl-7 oxo-4 naphtyridinyl-3] carbonyloxy acétate de n-nonyle.

8 - Dérivé selon la revendication 1 caractérisé en ce qu'il est constitué par l'[ethyl-1 oxo-4 méthylènedioxy-6,7 dihydro-1,4 quinolinyl-3] carbonyloxy acétate de n-nonyle.

9 - Procédé de préparation des dérivés selon les revendications 1 à 8 caractérisé en ce que l'on fait réagir un sel alcalin ou alcalino-terreux d'un dérivé de l'acide dihydro-1,4 ethyl-1 oxo-4 pyridine carboxylique-3 avec un ester d'un acide halogénoalkyl-carboxylique dans un solvant inerte.

10 - Médicament utilisable en médecine humaine et/ou vétérinaire caractérisé en ce qu'il contient au moins un composé suivant les revendications 1 à 8 à l'état pur ou en association avec un ou plusieurs adjuvants ou diluants compatibles et pharmaceutiquement acceptables.

11 - Antibactériens contenant ou constitués par au moins un produit selon la formule générale (I).

12 - Relaxants musculaires contenant ou constitués par au moins un produit selon la formule générale (I).

13 - Bronchodilatateurs contenant ou constitués par au moins un produit selon la formule générale (I).

14 - Antidépresseurs contenant ou constitués par au moins un produit selon la formule générale (I).

15 - Diurétiques contenant ou constitués par au moins un produit selon la formule générale (I).

REVENDICATIONS

1. Procédé de préparation des antibactériens à action pharmacologique multiple de formule générale I ci-après :

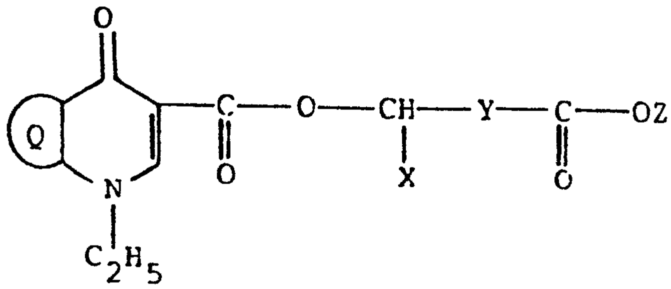

(I)

dans laquelle :

. Q représente un cycle aromatique éventuellement substitué ou un hétérocycle comprenant un ou deux atomes d'azote éventuellement substitué.

. X représente un atome d'hydrogène ou un groupement alkyle linéaire ou ramifié comprenant 1 à 6 atomes de carbone, un noyau phényl éventuellement substitué.

. Y représente une liaison ou un groupement alkyle linéaire ou ramifié comprenant 1 à 10 atomes de carbone.

. Z représente un atome d'hydrogène :

- ou un groupement alkyle linéaire ou ramifié contenant de 1 à 11 atomes de carbone et pouvant déterminer un cycle saturé constitué de 4 à 7 atomes de carbone.

- ou un groupement aralkyle comprenant de 7 à 12 atomes de carbone.

- ou un enchainement aliphatique de 1 à 4 méthylène lié à un hétérocycle à 5 ou 6 chainons, l'hétéroatome étant choisi parmi l'azote, le soufre, l'oxygène.

- ou un groupement hétéroalkyle comprenant de 1 à 10 atomes de carbone et l'hétéroatome étant choisi parmi l'azote, le soufre ou l'oxygène,

caractérisé en ce que l'on fait réagir un sel alcalin ou alcalino-terreux d'un dérivé de l'acide dihydro-1,4 éthyl-1

oxo-4 pyridine carboxylique-3 avec un ester d'un acide halogénoalkyl-carboxylique dans un solvant inerte.

2. Procédé de préparation selon la Revendication 1, caractérisé en ce l'on transforme le produit de formule générale I en sels d'addition avec les acides ou les bases pharmaceutiquement acceptables.

3. Procédé de préparation de dérivés de formule générale I selon la Revendication 1, caractérisé en ce que Q détermine avec la pyridone le dérivé éthyl-1 dihydro-1,4 méthylènedioxy-6,7 oxo-4 quinolyl-3.

4. Procédé de préparation de dérivés de formule générale I selon la Revendication 1, caractérisé en ce que Q détermine avec la pyridone le dérivé éthyl-1 dihydro-1,4 méthyl-7 oxo-4 naphtyridinyl-3.

5. Procédé selon la Revendication 1, caractérisé en ce que le produit obtenu est constitué par l'[éthyl-1 dihydro-1,4 méthyl-7 oxo-4 naphtytidinyl-3] carbonyloxy-4 butyrate d'éthyle.

6. Procédé selon la Revendication 1, caractérisé en ce que le produit obtenu est constitué par l'[éthyl-1 oxo-4 méthylènedioxy-6,7 dihydro-1,4 quinolinyl-3] carbonyloxy-4 butyrate d'éthyle.

7. Procédé selon la Revendication 1, caractérisé en ce que le produit obtenu est constitué par l'[éthyl-1 dihydro-1,4 méthyl-7 oxo-4 naphtyridinyl-3] carbonyloxy acétate de n-nonyle.

8. Procédé selon la Revendication 1, caractérisé en ce que le produit obtenu est constitué par l'[éthyl-1 oxo-4 méthylènedioxy-6,7 dihydro-1,4 quinolinyl-3] carbonyloxy acétate de n-nonyle.

Office européen des brevets

**0207845**

# RAPPORT DE RECHERCHE EUROPEENNE

Numéro de la demande

EP 86 40 1320

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl 4) |
|---|---|---|---|
| X | US-A-3 590 036 (G.Y. LESHER) * Revendication 1; colonne 10, lines 25-51; colonne 62, example 240 * --- | 1,10 | C 07 D 471/04<br>C 07 D 491/04<br>C 07 D 215/56<br>A 61 K 31/435<br>A 61 K 31/47<br>A 61 K 31/495//<br>(C 07 D 471/04<br>C 07 D 221:00<br>C 07 D 221:00)<br>(C 07 D 491/04<br>C 07 D 317:00<br>C 07 D 221:00) |
| P,A | EP-A-0 165 155 (PANMEDICA) * Revendication 1; page 13, lignes 17-33; page 14, lignes 1-16 * ----- | 1,10 | |
| | | | **DOMAINES TECHNIQUES RECHERCHES (Int Cl 4)** |
| | | | C 07 D 471/00<br>C 07 D 491/00<br>A 61 K 31/00 |

Le présent rapport de recherche a été établi pour toutes les revendications

| Lieu de la recherche | Date d achèvement de la recherche | Examinateur |
|---|---|---|
| LA HAYE | 17-09-1986 | ALFARO I. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82